# EUROPÄISCHE PATENTSCHRIFT

(11) **EP 0 879 052 B1**
(45) Veröffentlichungstag und Bekanntmachung des Hinweises auf die Patenterteilung: **20.12.2000**
(21) Anmeldenummer: 97901078.2
(22) Anmeldetag: 23.01.1997
(51) Int. Cl.: A61K 9/70, A61K 7/04, A61K 7/043

(54) **NAGELWACHSTUMSFÖRDERNDE ZUBEREITUNGEN**
PREPARATIONS STIMULATING NAIL GROWTH
PREPARATIONS STIMULANT LA POUSSE DES ONGLES

(30) Priorität: 06.02.1996 DE 19604190
(43) Veröffentlichungstag der Anmeldung: 25.11.1998
(73) Patentinhaber: Aventis Pharma Deutschland GmbH, 65929 Frankfurt am Main (DE)
(72) Erfinder: BOHN, Manfred, D-65719 Hofheim (DE); KRAEMER, Karl, Theodor, D-63225 Langen (DE); ULBRICHT, Horst, D-63599 Biebergemünd (DE)
(86) Internationale Anmeldenummer: EP9700311
(87) Internationale Veröffentlichungsnummer: WO9728790

(56) Entgegenhaltungen:
- EP-A- 0 353 896
- WO-A-87/02580
- WO-A-95/23597
- FR-A- 2 689 008
- US-A- 4 927 626

## Beschreibung

Die Erfindung betrifft einen Nagellack, enthaltend eine nagelwachstumsfördernde Verbindung und einen wasserunlöslichen Filmbildner, Verfahren zu dessen Herstellung und Verwendung derselben zur Behandlung von Wachstumsstörungen des Nagels.

Die den Nagel abschließende und schutzgebende Nagelplatte ist ein verhorntes, hartes Anhangsgebilde der Finger- oder Zehenspitzenhaut, das aus einer taschenartigen Einstülpung der Epidermis am Finger oder Zehenrücken hervorwächst.

Die Bildung des Nagelmaterials für die Nagelplatte erfolgt primär in der Nagelmatrix, einem spezialisierten Gewebe, das den unteren Anteil der Nageltasche von ihrem proximalen Ende bis zur Lunula einnimmt. Der Nagelmatrixregion schließt sich nach distal das Nagelbett an, mit dem die an ihrer Unterfläche längsleistenartig strukturierte Nagelplatte bis zum Hyponychium fest verhaftet ist. Als Hyponychium bezeichnet man den zwischen Nagelbett und Fingerbeere gelegenen dorsalen Epidermisbereich.

Die Oberfläche der Nagelplatte ist glatt, ihre Farbe infolge Durchscheinens korialer Kapillaren zart rosa. Nur ein am proximalen Ende vorhandener 1 - 5 mm großer halbmondförmiger Bezirk - die Lunula - erscheint weißlich.

Die Wachstumsgeschwindigkeit der Nagelplatte, d.h. seine Verlängerung über den freien Rand hinaus, ist abhängig vom Ausmaß der Neubildung von Nagelzellen in der Nagelmatrix. Das dort gebildete Zellmaterial differenziert sich zu plattenartigen Hornstrukturen, die passiv distalwärts vorgeschoben werden. Der Nagel wächst kontinuierlich während des ganzen Lebens. Dabei nimmt die Wachstumsrate im Alter ab. Die durchschnittliche wöchentliche Längenzunahme der Fingernägel beträgt 0,5 bis 1,2 mm, wobei neben dem Lebensalter und Geschlecht, Durchblutung, Ernährung sowie physiologische Belastung auf diesen Wert Einfluß nehmen können. So sollen die Nägel an der Arbeitshand schneller wachsen. Fußnägel wachsen insbesondere bei älteren Personen deutlich langsamer als Fingernägel.

Bei normaler Nagelwachstumsgeschwindigkeit eines etwa 35-jährigen beträgt die Zeit, die zum Herauswachsen eines neuen Fingernagels erforderlich ist, etwa 6 Monate, die Erneuerung eines Zehennagels erfolgt in etwa 12 Monaten.

Neben anlagenmäßigen Faktoren, die zum Unterbleiben des Nagelwachstums führen, können zahlreiche lokale oder allgemeine Krankheitsprozesse wie auch Einwirkungen durch Gifte, Medikamente, Chemikalien und Traumen das Wachstum und das Aussehen des Nagels negativ beeinflussen.

Eine ganz entscheidende Rolle spielt das Nagelwachstum bei der Behandlung und bei der erforderlichen Behandlungsdauer von Onychomykosen; unter Onychomykosen versteht man durch Pilze hervorgerufene Infektionen des Nagelbettes, die sich mit zunehmender Krankheitsdauer über den gesamten Nagel einschließlich der sichtbaren Nagelplattenanteil ausdehnen können.

Bei der distalen subungualen Onychomykose, dem am häufigsten zu beobachtenden klinischen Typus, befallen die Erreger das Nagelorgan, indem sie sich zunächst zu den hyponychialen Hornschichten Zutritt verschaffen. Im weiteren Verlauf der Infektion wird auch die Unterseite der Nagelplatte angegriffen. In späteren Stadien kommt es in der Nagelplatte zu Verfärbungen und Umstrukturierungen, wodurch sich das Aussehen des Nagels vollständig ändert. Keratophile Pilze wie Trichophyton rubrum oder T. mentagrophytes verändern die Nagelplatte in einer ganz charakteristischen Weise. Sie führen zu einer sichtbaren Verfärbung, die im fortschreitenden Stadium mit einer Zerstörung der Lamellenstruktur der Nagelplatte einhergeht und letzlich zu einer vollständigen Zerstörung des Schutzschildes führt. Mit Fortschreiten der Infektion bilden die subungualen Gewebstrümmer des Hyponychium und des Nagelbetts eine ideale Brutstätte für weitere Mikroorganismen, was wiederum zur zusätzlichen Veränderung der chemischen und physikalischen Eigenschaften der Nagelplattenunterseite beiträgt. Schließlich löst sich die Nagelplatte zunächst vom Nagelbett, was für den Patienten bei Bewegungen mit großen Schmerzen verbunden ist. So können die Betroffenen durch die Einwirkung der Pilze die gesamte Nagelplatte verlieren, so daß nur noch das hyperkeratotische Nagelbett übrig bleibt, das somit den äußeren Einflüssen schutzlos ausgeliefert ist. Zielgerichtete Bewegungen sind wegen des Fehlens der als Widerlager fungierenden Nagelplatte kaum mehr möglich.

Die Ausbreitungsgeschwindigkeit der Infektion ist von Fall zu Fall unterschiedlich. Sie hängt einerseits von der Wachstumsgeschwindigkeit der eindringenden Mikroorganismen vom freien Nagelende in Richtung Nagelmatrix, sowie andererseits von der Wachstumsrate des Nagels von der Nagelmatrix in Richtung freies Nagelende ab.

Nur ein gesunder Nagel ist aufgrund seiner Wachstumsgeschwindigkeit gegen ein Vordringen von Mikroorganismen vom freien Nagelende her geschützt. Läuft das Nagelwachstum jedoch alters- oder krankheitsbedingt verlangsamt ab, so können sich die Mikroorganismen bei Nichtbehandlung ungehindert ausbreiten.

Eine bekannte Therapie von Onychomykosen ist derzeit die topische Behandlung der erkrankten Nägel mit antimykotisch wirksamen Lackzubereitungen zum Teil in Kombination mit systemisch wirkenden Antimykotika. Ein Nachteil dieser Behandlungsmethode besteht darin, daß bis zur klinischen Heilung der erkrankten Nägel, d.h. bis die befallenen Nagelareale herausgewachsen sind und ein neuer Nagel sauber nachgewachsen ist, relativ lange behandelt werden muß. Dies führt dazu, daß die gewöhnlich vielmonatige Behandlung von Patienten vielfach nicht durchgehalten wird und daß dadurch der Therapieerfolg ausbleibt. Auch stellt die häufig angewandte topisch/systemische Kombinationstherapie aufgrund der Preissituation bei den systemischen Antimykotika einen erheblichen Kostenfaktor dar.

US 4,927,628 beschreibt die Verwendung von 6-Amino-1,2-dihydroxy-2-imino-4-piperidinpyrimidin (Minoxidil) zur Beschleuschleunigung des Nagelwachestums. Dazu werden topische Formulierungen vorgeschlagen, die auch Ethanol enthalten. Es wird kein Hinweis auf eine topische Formulierung gegeben, die einen wasserunlöslichen Nagellack enthält.

Es wurde nun gefunden, daß man das Wachstum von Nägeln beschleunigen kann, wenn man die erfindungsgemäße Zubereitung auf die Nägel, inbesondere auf die erkrankten Nägel aufträgt. Die Zubereitungen sind nicht nur als zusätzliche unterstützende Maßnahme zur Wachstumsbeschleunigung bei der spezifischen Therapie von Onychomykosen geeignet, sondern können auch zur Behandlung von Nagelwachstumsstörungen verschiedener Genese eingesetzt werden.

Die Erfindung betrifft daher einen Nagellack, enthaltend einen wasserunlöslichen Filmbildner und mindestens eine vasodilatatorisch wirksame Verbindung gemäß Anspruch 1, dessen Herstellung gemäß Anspruch 8 und die Verwendung des Nagellacks zur Hertellung eines Arzneimittels zur Behandlung von Wachestumsstörungen des Nagels gemäß Anspruch 9.

Verbindungen, die eine vasodilatatorische Wirkung haben, sind beispielsweise
1. Verbindungen der Formel I 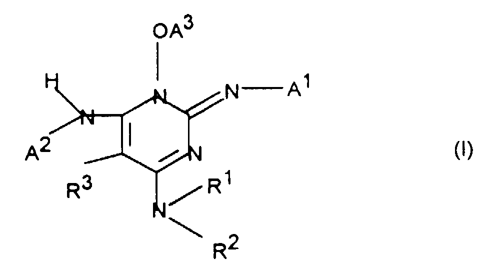 wobei
   R¹ und R² unabhängig voneinander für
      1) Wasserstoffatom,
      2) (C₁-C₈)-Alkyl,
      3) (C₂-C₈)-Alkenyl,
      4) Phenyl-(C₁-C₈)-Alkyl,
      5) Naphthyl-(C₁-C₄)-Alkyl oder
      6) (C₃-C₈)-Cycloalkyl stehen,
      7) R¹ und R² zusammen mit dem N-Atom einen Heterozyklus bilden aus der Gruppe
         7.1 Aziridinyl,
         7.2 Azetinyl,
         7.3 Pyrrolidinyl,
         7.4 Piperidinyl,
         7.5 Hexahydroazepinyl,
         7.6 Heptamethylimino,
         7.7 Octamethylimino,
         7.8 Morpholinyl oder
         7.9 4-(C₁-C₄)-Alkyl-piperazinyl, oder
      8) ein Rest aus 7), wobei die C-Atome vom Heterozyklus durch 1 bis 3 (C₁-C₄)-Alkylreste substituiert sind,
   R³ für
      1) Wasserstoffatom,
      2) (C₁-C₈)-Alkyl,
      3) (C₂-C₈)-Alkenyl,
      4) Phenyl-(C₁-C₈)-Alkyl,
      5) Naphthyl-(C₁-C₈)-Alkyl,
      6) Benzyl,
      7) Phenyl,
      8) Naphthyl,
      9) (C₃-C₈)-Cycloalkyl oder
      10) (C₁-C₆)-Alkyl, ein- oder mehrfach substituiert durch Halogen, steht, und
   A¹, A² und A³ unabhängig voneinander für Wasserstoffatom oder Acetyl stehen.

   Bevorzugt ist eine Verbindung der Formel I, wobei
   R¹ und R² zusammen mit dem N-Atom den Heterozyklus Piperidinyl bilden,
   R³ für Wasserstoffatom steht, und
   A¹, A² und A³ unabhängig voneinander für Wasserstoffatom oder Acetyl stehen.

   Insbesondere bevorzugt ist eine Verbindung der Formel I, wobei
   R¹ und R² zusammen mit dem N-Atom den Heterozyklus Piperidinyl bilden und
   R³, A¹, A² und A³ für Wasserstoffatom stehen,
2. Verbindungen wie Dihydralazin, Diisopropylamin oder Diazoxid,
3. Calciumantagonisten wie Nifedipin, Nicardipin, Verapamil, Diltiazem, Nisoldipin, Nitrendipin, Nivaldipin, Isradipin, Felodipin, Nimodipin, Gallopamil, Fendilin, Flunarizin, Amlodipin, Diperdipin, Fluspirilen, Primozid, Fantofaron, Nicergolin oder Clycandelat.
4. Angiotensin-Converting-Enzym-Hemmer wie Quinapril, Lisinopril, Benzazepril, Captopril, Ramipril, Fosinopril, Cifazapril oder Trandolapril.
5. Methylxanthinverbindungen wie Pentoxifyllin, Propentofyllin oder Torbafyllin.
6. Haarwachstumfördernde Verbindungen wie innere Salze von 2,4-Diamino-6-alkoxy-3-sulfoxypyrimidinhydroxid mit 1 bis 6 Kohlenstoffatomen im Alkoxyrest wie beschrieben in EP 0 427 625 oder das innere Salz von 2,4-Diamino-6-butoxy-3-sulfoxypyrimidinhydroxid; Pyridin-1-oxid-derivate wie beschrieben in WO 92 21317 oder 2,6-Diamino-4-piperidinopyridin; 2,6-Diamino-1,3,5-triazin-derivate wie beschrieben in WO 91 19701 oder 2,6-Diamino-4-butoxy-1,3,5-triazin-1-oxid.

Die Herstellung der Verbindung der Formel I erfolgt wie in U.S. 3,461,461 beschrieben.

Mit dem erfindungsgemäßen Nagellack als zusätzliche unterstützende Maßnahme zur Wachstumsbeschleunigung läßt sich bei der spezifischen Therapie von Onychomykosen die Behandlungszeit wesentlich verkürzen, was neben einer Therapiekostenoptimierung zu einer erheblichen Verbesserung der Patientencompliance beiträgt. Im Hinblick auf die bisherigen schlechten Therapieerfahrungen infolge mangelnder Compliance der Patienten, für die der Heilungserfolg nicht schnell genug sichtbar wird, ist dies ein überaus wichtiger Befund.

Der nach dem Trocknen der Lackzubereitung vorliegende wasserunlösliche Lackfilm hat darüber hinaus gegenüber hydrophilen Systemen den Vorteil, daß er beim Waschen, Baden oder Duschen nicht von der Nageloberfläche entfernt wird, also danach nicht wieder neu aufgetragen werden muß. Er verhindert ferner, daß beim Manipulieren mit Wasser bereits in den Nagel eingedrungener Wirkstoff wieder herausgelöst wird.

Die Behandlung von distalen subungualen Onychomykosen im Frühstadium ist mit dem erfindungsgemäßen Nagellack auch ohne zusätzlich spezifische antimykotische Therapie möglich.

Die Anwendung des erfindungsgemäßen Nagellacks ist nicht ausschließlich auf die Therapie oder auf eine zusätzliche Maßnahme bei der Therapie von Onychomykosen beschränkt. Der erfindungsgemäße Nagellack kann auch zur Behandlung von Nagelwachstumsstörungen verschiedenen anderen Ursprungs eingesetzt werden.

Der Gehalt an Wirkstoff in dem erfindungsgemäßen Nagellack ist von der Struktur eines jeden Wirkstoffs und damit von dessen Freigabe aus dem Lackfilm und seinem Penetrationsverhalten im Nagel abhängig.

In dem erfindungsgemäßen Nagellack, also der Lösemittel enthaltenden Anwendungsform, ist der Wirkstoff im allgemeinen in einer Menge von 0,1 bis 10, vorzugsweise von 2 bis 5 Gewichtsprozent, enthalten.

Die erfindungsgemäßen Nagellacke enthalten außer dem in einem Lösemittel oder Lösemittelgemisch gelösten Wirkstoff als notwendige Bestandteile noch einen oder mehrere Filmbildner, die nach dem Trocknen der Zubereitung einen wasserunlöslichen Film auf dem Nagel bilden.

Als Filmbildner eignen sich beispielsweise Stoffe auf der Basis von Cellulosenitrat oder physiologisch unbedenkliche Polymerisate wie sie in Kosmetika üblich sind, vorzugsweise als Gemisch mit Cellulosenitrat. Genannt seien beispielsweise Polyvinylacetat und partiell verseiftes Polyvinylacetat, Mischpolymerisate aus Vinylacetat einerseits und Acrylsäure oder Crotonsäure oder Maleinsäuremonoalkylester andererseits, ternäre Mischpolymerisate aus Vinylacetat einerseits und Crotonsäure und Vinylneodecanoat, oder Crotonsäure und Vinylpropionat andererseits, Mischpolymerisate aus Methylvinylether und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, insbesondere mit einem Gehalt an quartären Ammoniumgruppen, oder Polymere, Copolymere oder Mischungen, enthaltend Ethylacrylat, Methylmethacrylat oder Trimethylammonioethylmethacrylat-chlorid, oder Polyvinylacetale und Polyvinylbutyrale, alkylsubstituierte Poly-N-vinylpyrrolidone, Alkylester aus Mischpolymerisaten aus Olefinen und Maleinsäureanhydrid und Umsetzungsprodukte von Kolophonium mit Acrylsäure. In den Estern sind die Alkylreste gewöhnlich kurzkettig und haben meistens nicht mehr als vier C-Atome.

Als physiologisch unbedenkliche Lösemittel kommen Stoffe wie in Kosmetika übliche Kohlenwasserstoffe, halogenierte Kohlenwasserstoffe, Alkohole, Ether, Ketone und Ester in Betracht, insbesondere Essigsäureester von einwertigen Alkoholen wie Ethyl- und Butylacetat, gegebenenfalls im Gemisch mit aromatischen Kohlenwasserstoffen wie Toluol und/oder Alkoholen wie Ethanol oder Isopropanol.

Die Kombination der Lösemittel ist bekanntlich von entscheidender Bedeutung für die Trockenzeit, Verstreichbarkeit und andere wichtige Eigenschaften des Lackes oder des Lackfilms. Das Lösemittelsystem besteht vorzugsweise aus einer optimalen Mischung aus Niedrigsiedern (= Lösemittel mit einem Siedepunkt von 100°C) und Mittelsiedern ( =Lösemittel mit einem Siedepunkt bis 150°C), gegebenenfalls mit einem kleinen Anteil Hochsiedern (=Lösemittel mit einem Siedepunkt bis 200°C).

Die erfindungsgemäßen Nagellacke können weiterhin in Kosmetika gebräuchliche Zusätze erhalten wie Weichmacher auf Phthalat- Glyceryltriacetat- oder Campherbasis, Farbstoffe oder Farbpigmente, Perlglanzmittel, Sedimentationsverzögerer, Sulfonamidharze, Silikate, Riechstoffe, Netzmittel wie Natriumdioctylsulfo-succinat, Lanolinderivate, Lichtschutzmittel wie 2-Hydroxy-4-methoxybenzo-phenon, antibakteriell wirksame Substanzen und Stoffe mit keratolytischer und/oder keratoplastischer Wirkung wie Ammoniumsulfit, Ester und Salze der Thioglykolsäure, Harnstoff, Allantoin, Enzyme und Salizylsäure.

Gefärbte oder pigmentierte Nagellacke haben beispielsweise den Vorteil, daß die erfindungsgemäße Zubereitung dem Schönheitsempfinden des Patienten angepaßt werden kann und die derzeit bestehenden Nagelveränderungen für Dritte nicht unmittelbar sichtbar sind.

Das erfindungsgemäße Verfahren zur Herstellung des Nagellacks besteht darin, daß man den wasserunlöslichen Filmbildner in gelöster Form mit dem Wirkstoff oder Wirkstoffen mischt und die Zubereitung soweit erforderlich weiter verarbeitet.

Die Erfindung betrifft ferner einen Nagellack, enthaltend
1. einen wasserunlöslichen Filmbildner,
2. eine vasodilatatorische Verbindung und
3. ein topisch wirksames Antimykotikum.

Topisch wirksame Antimykotika sind Hydroxypyridone wie Ciclopirox, Piroctone oder Rilopirox, Morpholinderivate wie Amorofine, Azole wie Bifonazol, Clotrimazol, Econazol, Miconazol, Oxiconazol, Croconazol, Fenticonazol, Tioconazol, Ketoconazol oder Isoconazol oder Allylverbindungen wie Terbinafin oder Naftifin sowie Griseofulvin, Tolciclate, Tolnaftat und Butenafin.

Als geeignete Hydroxypyridone seien beispielsweise ferner genannt: 1-Hydroxy-4-methyl-6-n-hexyl-6-iso-hexyl-, -6-n-heptyl- oder -6-iso-heptyl-2-pyridon, 1-Hydroxy-4-methyl-6-octyl- oder -6-iso-octyl-2-pyridon, insbesondere das 1-Hydroxy-4-methyl-6-(2,4,4-trimethylpentyl)-2-pyridon, 1-Hydroxy-4-methyl-6-cyclohexyl-2-pyridon, 1-Hydroxy-4-methyl-6-cyclohexylmethyl- oder-6-cyclohexylethyl-2-pyridon, wobei der Cyclohexylrest jeweils auch einen Methylrest tragen kann, 1-Hydroxy-4-methyl-6-(2-bicyclo[2,2,1]heptyl)-2-pyridon, 1-Hydroxy-3,4-dimethyl-6-benzyl- oder -6-dimethylbenzyl-2-pyridon und 1-Hydroxy-4-methyl-6-(β-phenyl-ethyl)-2-pyridon.

Der Gehalt an wasserunlöslichen Filmbildner, vasodilatatorischer Verbindung, Lösemitteln und weiteren Zusatzstoffen entspricht dem des obengenannten Nagellacks ohne Antimykotikum.

Der Gehalt an topisch wirksamen Antimykotika in dem erfindungsgemäßen Nagellack ist von der Struktur des jeweiligen Antimykotikums und von deren Freigabe aus dem Lackfilm, seinem Penetrationsverhalten im Nagel und seinen antimikrobiellen Eigenschaften abhängig.

In dem erfindungemäßen Nagellack, der Lösemittel enthaltenden Anwendungsform, ist das topisch wirksame Antimykotikum im allgemeinen in einer Menge von 0,5 bis 20, vorzugsweise 2 bis 15 Gewichtsprozent (Gew.-%) enthalten. Der Mindestgehalt in medizinischen Nagellacken zur Behandlung von Mykosen beträgt 4 Gew.-%; die zur Prophylaxe verwendeten Nagellacke enthalten weniger als 4 und mindestens 1 Gew.-% des Antimykotikums. Jeweils bezogen auf die Menge der nicht flüchtigen Bestandteile, das ist die Summe der Filmbildner, vasodilatatorische Verbindung, der gegebenenfalls vorhandene Pigmente, Weichmacher und anderen nicht flüchtigen Zusätze sowie des topisch wirksamen Antimykotikums, ist in den erfindungsgemäßen Nagellacken das Antimykotikum im allgemeinen in einer Menge von 2 bis 80, vorzugsweise von 10 bis 60 und insbesondere von 20 bis 40 Gew.-% enthalten.

Die Erfindung betrifft ferner die Verwendung der erfindungsgemäßen Nagellackzubereitung zur Herstellung eines Arzneimittels zur Behandlung von Wachstumsstörungen des Nagels.

### Beispiel 1

Eine erfindungsgemäße Zubereitung weist folgende Zusammensetzung auf:

| | |
|---|---|
| 6-Amino-4-piperidino-1,2-dihydro-1-hydroxy-2-iminopyrimidin | 2,5 % |
| Ethylacrylat-Methylmethacrylat-Trimethylammonioethyl-methacrylat-chlorid in einem Molverhältnis von 1:2:0,2 (siehe Überzogene Arzneiformen, Autoren Bauer, Lehmann, Osterwald und Rothgang, Seiten 239-242, Wiss. Verlagsgesellschaft mbH, Stuttgart, 1988; EUDRAGIT® RL 100) | 7,0 % |
| Ethanol 96 % | 75,0 % |
| Ethylacetat | 10,5 % |
| Butylacetat | 5,0 % |

Die prozentualen Mengenangaben sind auf das Gewicht bezogen. Der Nagellack wird durch Lösen der verschiedenen Komponenten in den Lösemitteln hergestellt.

Die Wirkung der erfindungsgemäßen Zubereitungen wird in Permeationstests an Kuhhornplättchen und in Behandlungsversuchen an Probanden nachgewiesen. Der Permeationstest an Kuhhornplättchen erlaubt es, die Freigabe eines Wirkstoffes aus einer bestimmten Zubereitung und die sich daran anschließende Permeation durch Keratinmaterial zu prüfen.

Als Kontrollbeispiel wird

| | |
|---|---|
| 6-Amino-4-piperidino-1,2-dihydro-1-hydroxy-2-iminopyrimidin | 2,5 % |
| in Ethanol 96 % | 97,5 % |

gelöst.

### A) Permeationstest an Kuhhornplättchen

Die Messung der Wirkstoffpermeation wird mittels zeitaufgelöster Fourier-Transform-Infrarotspektroskopie (siehe Th. M. Bayerl et al.; J. Invest. Dermatol. 105:291-295, 1995) ATR-Technik durchgeführt:

Auf die Oberseite eines Kuhhornplättchen von 0,5 mm Dicke werden 100 µl der Prüfzubereitung (erfindungsgemäße Zubereitung oder Kontrollbeispiel) aufgetragen. Das Kuhhornplättchen wird mit seiner Unterseite auf der Oberseite eines Siliciumkristalls fixiert. Nach der Permeation des Wirkstoffes durch das Kuhhornplättchen wird das FT-IR-Spektrum der Substanz in Abhängigkeit von der vorhandenen Wirkstoffkonzentration beeinflußt, so daß durch Vergleichsmessungen mit der Prüfzubereitung, die direkt auf den Meßkristall aufgetragen wird, quantitative Aussagen über die permeierte Wirkstoffmenge in Abhängigkeit von der Zeit möglich sind.

Die Versuche zeigen, daß die erfindungsgemäße Nagellackzubereitung eine mehr als zehnfache Permeationsrate des Wirkstoffes durch Keratinmaterial zeigt, im Vergleich mit dem Kontrollbeispiel. Dies ist ein überraschender Befund, da nicht vorauszusehen war, daß der Wirkstoff, aus dem nach dem Trocknen der Lackzubereitung vorliegenden wasserunlöslichen Feststoffsystem, besser bioverfügbar ist als aus der ethanolischen Lösung.

### B) Wirksamkeitsprüfung

Die nagelwachstumsfördernden Eigenschaften der erfindungsgemäßen Nagellackzubereitung ist an 2 Menschen überprüft worden. Als direkter Wachstumsvergleich dienten die unbehandelten Finger- und Fußnägel der jeweils anderen Hand oder des anderen Fußes. Um speziell bei den Händen eine Fehlinterpretation wegen eines möglicherweise schnelleren Wachstums der Nägel an der Arbeitshand aufgrund der dort vorherrschenden besseren Durchblutung des Nagelorgans mit zu untersuchen oder auszuschließen, werden bei einer Testperson die Fingernägel der Arbeitshand und bei der anderen Testperson die Fingernägel der Nichtarbeitshand mit der Prüfzubereitung behandelt. Daher werden bei einer Testperson die Nägel der rechten Arbeitshand und die Nägel des linken Fußes mit der Prüfsubstanz behandelt, während das Auftragen der Zubereitung bei der anderen Testperson spiegelbildlich erfolgt.

Zur Bestimmung der Wachstumsgeschwindigkeit wird die Länge aller Nägel mit einer Präzisionsschieblehre ermittelt, wobei der proximale Ausgangspunkt der Messung vom Zenit der Lunula erfolgt, da dieser Punkt einen Fixpunkt darstellt. Als distale Begrenzung wird der Nagelrand gewählt.

Ergebnisse: (Zunahme des Längenwachstum vom behandelten gegen den unbehandelten Nagel; Behandlungszeit 4 Wochen einmal täglich)

| Fingernägel | |
|---|---|
| Behandlung Arbeitshand | + 45,3 % |
| Behandlung Nichtarbeitshand | + 18,6 % |

| Fußnägel | |
|---|---|
| Behandlung linker Fuß | + 27,6 % |
| Behandlung rechter Fuß | + 23,9 % |

Zusätzlich zur Längenwachstumszunahme wird die Flächenwachstumszunahme der Nägel mittels einer durchsichtigen Millimeterfolie ermittelt.

Ergebnisse: (Zunahme des Flächenwachstum vom behandelten gegen den unbehandelten Nagel; Behandlungszeit 4 Wochen einmal täglich)

| Fingernägel | |
|---|---|
| Behandlung Arbeitshand | + 53,3 % |
| Behandlung Nichtarbeitshand | + 45,7 % |

| Fußnägel | |
|---|---|
| Behandlung linker Fuß | + 110,8 % |
| Behandlung rechter Fuß | + 177,5 % |

### Beispiel 3

Eine erfindungsgemäße Zubereitung weist folgende Zusammensetzung auf:

| | |
|---|---|
| 6-Amino-4-piperidino-1,2-dihydro-1-hydroxy-2-iminopyrimidin | 2,0 % |
| 4-[3-[p-(1,1-dimethylpropyl)-phenyl]2-methyl-propyl]-2,6-dimethyl-morpholinhydrochlorid | 5,0 % |
| EUDRAGIT® RL 100 | 10,0 % |
| Ethanol 96 % | 73,0 % |
| Ethylacetat | 10,0 % |

### Beispiel 4

Eine erfindungsgemäße Zubereitung weist folgende Zusammensetzung auf:

| | |
|---|---|
| 4-Amino-4-piperidino-1,2-dihydro-1-hydroxy-2-iminopyrimidin | 2,5 % |
| 1-Hydroxy-4-methyl-6-cyclohexyl-2-pyridon | 8,0 % |
| 50 %ige Lösung eines Mischpolymerisates aus Methylvinylether und Maleinsäuremonobutylester in Isopropanol | 35,0 % |
| Ethanol 96 % | 44,5 % |
| Ethylacetat | 10,0 % |

### Beispiel 5

Eine erfindungsgemäße Zubereitung weist folgende Zusammensetzung auf:

| | |
|---|---|
| 5-[(3,4-Dimethyl-oxyphenethyl)methylamino]-2-(3,4-dimethyloxyphenyl)-2-isopropylvaleronitrile-hydrochlorid (Verapamilhydrochlorid) | 2,0 % |
| 1-[2,4-Dichloro-β-(2,4-dichlorobenzyloxy)phenethyl]imidazol (Miconazol) | 2,0 % |
| Polyvinylbutyral | 3,8 % |
| Cellulosenitrat | 3,1 % |
| Dibutylphthalat | 0,6 % |
| Ethylacetat | 10,0 % |
| Ethanol 96 % | 78,5 % |

### Beispiel 6

Eine erfindungsgemäße Zubereitung weist folgende Zusammensetzung auf:

| | |
|---|---|
| Angiotensin-Converting-Enzym-Hemmer in Kombination mit Allylantimykotikum (2S,3aS,6aS)-1-[(S)-N-[(S)-1-Ethoxycarbonyl-3-phenylpropyl]alanyl]octahydrocyclopenta[b]pyrrol-2-carbonsäure (Ramipril) | 2,0 % |
| (E)-N-(6,6-dimethyl-2-hepten-4-inyl)-N-methyl-1-nephthylmethylamin (Terbinafin) | 0,5 % |
| Methacrylsäure-Ethylacrylat-1:1-Copolymer | 6,5 % |
| Ethanol 96 % | 71,0 % |
| Ethylacetat | 20,0 % |

## Patentansprüche

1. Nagellack, enthaltend einen wasserunlöslichen Filmbildner und eine vasodilatatorisch wirksame Verbindung der Formel I, 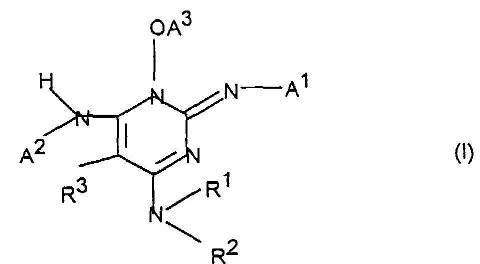 wobei
R¹ und R² unabhängig voneinander für
1) Wasserstoffatom,
2) (C₁-C₈)-Alkyl,
3) (C₂-C₈)-Alkenyl,
4) Phenyl-(C₁-C₈)-Alkyl,
5) Naphthyl-(C₁-C₄)-Alkyl oder
6) (C₃-C₈)-Cycloalkyl stehen oder
7) R¹ und R² zusammen mit dem N-Atom einen Heterozyklus bilden aus der Gruppe
7.1 Aziridinyl,
7.2 Azetinyl,
7.3 Pyrrolidinyl,
7.4 Piperidinyl,
7.5 Hexahydroazepinyl,
7.6 Heptamethylimino,
7.7 Octamethylimino,
7.8 Morpholinyl oder
7.9 4-(C₁-C₄)-Alkyl-piperazinyl, oder
8) ein Rest aus 7), wobei die C-Atome vom Heterozyklus durch 1 bis 3 (C₁-C₄)-Alkylreste substituiert sind,
R³ für
1) Wasserstoffatom,
2) (C₁-C₈)-Alkyl,
3) (C₂-C₈)-Alkenyl,
4) Phenyl-(C₁-C₈)-Alkyl,
5) Naphthyl-(C₁-C₈)-Alkyl,
6) Benzyl,
7) Phenyl,
8) Naphthyl,
9) (C₃-C₈)-Cycloalkyl oder
10) (C₁-C₆)-Alkyl, ein- oder mehrfach substituiert durch Halogen, steht, und
A¹, A² und A³ unabhängig voneinander für Wasserstoffatom oder Acetyl stehen, oder
Dihydralazin, Diisopropylamin, Diazoxid, Nifedipin, Nicardipin, Diltiazem, Nisoldipin, Nitrendipin, Nivaldipin, Isradipin, Felodipin, Nimodipin, Gallopamil, Fendilin, Flunarizin,Amlodipin, Diperdipin, Fluspirilen, Primozid, Fantofaron, Nicergolin, Cyclandelat, Quinapril, Lisinopril, Benzazepril, Captopril, Ramipril, Fosinopril, Cifazapril, Trandolapril, Pentoxifyllin, Propentofyllin, Torbafyllin, inneres Salz von 2,4-Diamino-6-butoxy-3-sulfoxypyrimidinhydroxid, 2,6-Diamino-4-piperidinopyridin, 2,6-Diamino-4-butoxy-1,3,5-triazin-1-oxid oder eine Mischung davon.

2. Nagellack nach Anspruch 1, enthaltend die vasodilatatorisch wirksame Verbindung 6-Amino-4-piperidino-1,2-dihydro-1-hydroxy-2-iminopyrimidin.

3. Nagellack nach Anspruch 1, enthaltend als wasserunlöslicher Filmbildner ein Copolymerisat aus Ethylacrylat-Methylmethacrylat-Trimethylammonioethylmethacrylat-chlorid, Mischpolymerisat aus Methylvinylether und Maleinsäuremonobutylester, Polymerisat aus Polyvinylbutyral und Cellulosenitrat oder ein Copolymer aus Methacrylsäure und Ethylacrylat.

4. Nagellack nach Anspruch 1, enthaltend die vasodilatatorisch wirksame Verbindung in einer Menge von 0,1 bis 10 Gewichtsprozent, vorzugsweise 2 bis 5 Gewichtsprozent.

5. Nagellack nach einem oder mehreren der Ansprüche 1 bis 4, zusätzlich enthaltend ein topisch wirksames Antimykotikum.

6. Nagellack nach Anspruch 5, enthaltend als topisch wirksames Antimykotikum ein Hydroxypyridon wie Ciclopirox, Piroctone oder Rilopirox, Morpholinderivat wie Amorofine, Azol wie Bifonazol, Clotrimazol, Econazol, Miconazol, Oxiconazol, Croconazol, Fenticonazol, Tioconazol, Ketoconazol oder Isoconazol oder eine Allylverbindung wie Terbinafin oder Naftifin, Griseofulvin, Tolciclate, Tolnaftat oder Butenafin.

7. Nagellack nach Anspruch 5 oder 6, enthaltend das topisch wirksame Antimykotikum in einer Menge von 0,5 bis 20 Gewichtsprozent, bevorzugt von 2 bis 5 Gewichtsprozent.

8. Verfahren zur Herstellung eines Nagellacks gemäß einem oder mehreren der Ansprüche 1 bis 8, dadurch gekennzeichnet, daß man den wasserunlöslichen Filmbildner in gelöster Form mit der vasodilatatorisch wirksamen Verbindung mischt und gegebenenfalls das topisch wirksame Antimykotikum und weitere Zusätze hinzufügt.

9. Verwendung des Nagellacks gemäß einem oder mehreren der Ansprüche 1 bis 7 oder eines Nagellackes gemäß einem oder mehreren der Ansprüche 1 bis 7, enthaltend als vasodilatatorisch wirksame Verbindung die Verbindung Verapamil, zur Herstellung eines Arzneimittels zur Behandlung von Wachstumsstörungen des Nagels.

## Claims

1. A nail varnish comprising a water-insoluble film-forming agent and a compound having a vasodilating action of the formula I 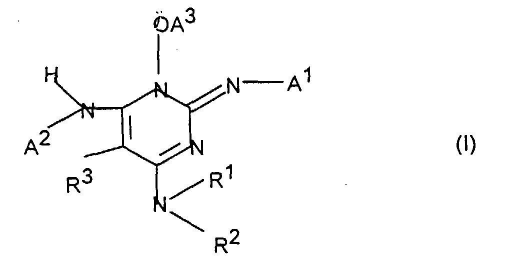 in which
R¹ and R² independently of one another are
1) a hydrogen atom,
2) (C₁-C₈)-alkyl,
3) (C₂-C₈)-alkenyl,
4) phenyl-(C₁-C₈)alkyl,
5) naphthyl-(C₁-C₄)-alkyl or
6) (C₃-C₈)-cycloalkyl, or
7) R¹ and R², together with the N atom, form a heterocyclic radical from the group consisting of
7.1 aziridinyl,
7.2 azetinyl,
7.3 pyrrolidinyl,
7.4 piperidinyl,
7.5 hexahydroazepinyl,
7.6 heptamethylimino,
7.7 octamethylimino,
7.8 morpholinyl or
7.9 4-(C₁-C₄)-alkyl-piperazinyl, or
8) a radical from 7), in which the carbon atoms of the heterocyclic radical are substituted by 1 to 3 (C₁-C₄)-alkyl radicals,
R³ is
1) a hydrogen atom,
2) (C₁-C₈)-alkyl,
3) (C₂-C₈)-alkenyl,
4) phenyl-(C₁-C₈)-alkyl,
5) naphthyl-(C₁-C₈)-alkyl,
6) benzyl,
7) phenyl,
8) naphthyl,
9) (C₃-C₈)-cycloalkyl or
10) (C₁-C₆)-alkyl, which is mono- or polysubstituted by halogen, and
A¹, A² and A³ independently of one another are a hydrogen atom or acetyl, or
dihydralazine, diisopropylamine, diazoxide, nifedipine, nicardipine, diltiazem, nisoldipine, nitrendipine, nivaldipine, isradipine, felodipine, nimodipin, gallopamil, fendiline, flunarizine, amlodipine, diperdipine, fluspirilene, primozid, fantofaron, nicergoline, cyclandelate, quinapril, lisinopril, benzazepril, captopril, ramipril, fosinopril, cifazapril, trandolapril, pentoxifylline, propentofylline, torbafylline, inner salt of 2,4-diamino-6-butoxy-3-sulfoxypyrimidine hydroxide, 2,6-diamino-4-piperidinopyridine, 2,6-diamino-4-butoxy-1,3,5-triazine 1-oxide or a mixture thereof.

2. A nail varnish as claimed in claim 1, comprising the compound having a vasodilating action 6-amino-4-piperidino-1,2-dihydro-1-hydroxy-2-iminopyrimidine.

3. A nail varnish as claimed in claim 1, comprising a copolymer of ethyl acrylate/methyl methacrylate/trimethylammonioethyl methacrylate chloride, a copolymer of methyl vinyl ether and maleic acid monobutyl ester, a polymer of polyvinylbutyral and cellulose nitrate or a copolymer of methacrylic acid and ethyl acrylate as the water-insoluble film-forming agent.

4. A nail varnish as claimed in claim 1, comprising the compound having a vasodilating action in an amount of 0.1 to 10 percent by weight, preferably 2 to 5 percent by weight.

5. A nail varnish as claimed in one or more of claims 1 to 4, additionally comprising an antimycotic having a topical action.

6. A nail varnish as claimed in claim 5, comprising a hydroxypyridone, such as ciclopirox, piroctone or rilopirox, a morpholine derivative, such as amorofine, an azole, such as bifonazole, clotrimazole, econazole, miconazole, oxiconazole, croconazole, fenticonazole, tioconazole, ketoconazole or isoconazole, or an allyl compound, such as terbinafin or naftifin, griseofulvin, tolciclate, tolnaftate or butenafin, as the antimycotic having a topical action.

7. A nail varnish as claimed in claim 5 or 6, comprising the antimycotic having a topical action in an amount of 0.5 to 20 percent by weight, preferably 2 to 5 percent by weight.

8. A process for the preparation of a nail varnish as claimed in one or more of claims 1 to 7, which comprises mixing the water-insoluble film-forming agent, in dissolved form, with the compound having a vasodilating action and, if appropriate, adding the antimycotic having a topical action and further additives.

9. The use of the nail varnish as claimed in one or more of claims 1 to 7 or of a nail varnish as claimed in one or more of claims 1 to 7, comprising the compound verapamil as the compound having a vasodilating action, for the preparation of a medicament for treatment of growth disturbances of the nail.

## Revendications

1. Vernis à ongles, contenant un agent filmogène insoluble dans l'eau et un composé à action vasodilatatrice de formule I, 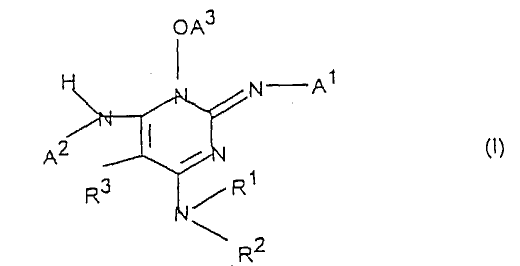 dans laquelle
R¹ et R² représentent, indépendamment l'un de l'autre,
1) un atome d'hydrogène,
2) un groupe alkyle en C₁-C₈,
3) un groupe alcényle en C₂-C₈,
4) un groupe phényl-alkyle(C₁-C₈),
5) un groupe naphtyl-alkyle(C₁-C₄),
6) un groupe cycloalkyle en C₃-C₈,
7) R¹ et R² forment ensemble, avec l'atome d'azote, un hétérocycle choisi dans l'ensemble constitué par les radicaux
7.1 aziridinyle,
7.2 azétinyle,
7.3 pyrrolidinyle,
7.4 pipéridinyle,
7.5 hexahydroazépinyle,
7.6 heptaméthylimino,
7.7 octaméthylimino,
7.8 morpholinyle et
7.9 4-alkyl(C₁-C₄)-pipérazinyle, ou
8) un radical choisi parmi ceux en 7), dans lequel les atomes de carbone de l'hétérocycle sont substitués par 1 à 3 groupes alkyle en C₁-C₄,
R³ représente
1) un atome d'hydrogène,
2) un groupe alkyle en C₁-C₈,
3) un groupe alcényle en C₂-C₈,
4) un groupe phényl-alkyle(C₁-C₈),
5) un groupe naphtyl-alkyle(C₁-C₈),
6) le groupe benzyle,
7) le groupe phényle,
8) le groupe naphtyle,
9) un groupe cycloalkyle en C₃-C₈ ou
10) un groupe alkyle en C₁-C₆ une ou plusieurs fois substitué par un ou des atomes d'halogène, et
A¹, A² et A³ représentent, indépendamment les uns des autres, un atome d'hydrogène ou le groupe acétyle, ou
de la dihydralazine, de la diisopropylamine, du diazoxide, de la nifédipine, de la nicardipine, du diltiazem, de la nisoldipine, de la nitrendipine, de la nivaldipine, de l'isradipine, de la félodipine, de la nimodipine, du gallopamil, de la fendiline, de la flunarizine, de l'amlodipine, de la diperdipine, du fluspirilène, du primozide, de la fantofarone, de la nicergoline, du clycandélate, du quinapril, du lisinopril, du benzazépril, du captopril, du ramipril, du fosinopril, du cifazapril, du trandolapril, de la pentoxifylline, de la propentofylline, de la torbafylline, du sel interne d'hydroxyde de 2,4-diamino-6-butoxy-3-sulfoxypyrimidine, de la 2,6-diamino-4-pipéridinopyridine, du 1-oxyde de 2,6-diamino-4-butoxy-1,3,5-triazine, ou un mélange de ceux-ci.

2. Vernis à ongles selon la revendication 1, contenant le composé à action vasodilatatrice 6-amino-4-pipéridino-1,2-dihydro-1-hydroxy-2-iminopyrimidine.

3. Vernis à ongles selon la revendication 1, contenant en tant qu'agent filmogène insoluble dans l'eau un copolymère d'acrylate d'éthyle/méthacrylate de méthyle/chlorure-méthacrylate de triméthylammonioéthyle, un copolymère d'éther méthylvinylique et de maléate de monobutyle, un polymère de poly(butyral de vinyle) et nitrate de cellulose ou un copolymère d'acide méthacrylique et d'acrylate d'éthyle.

4. Vernis à ongles selon la revendication 1, contenant le composé à action vasodilatatrice en une proportion de 0,1 à 10 % en poids, de préférence de 2 à 5 % en poids.

5. Vernis à ongles selon une ou plusieurs des revendications 1 à 4, contenant en outre un agent antimycosique à action locale.

6. Vernis à ongles selon la revendication 5, contenant en tant qu'agent antimycosique à action locale une hydroxypyridone telle que le ciclopirox, la piroctone ou le rilopirox, un dérivé de la morpholine tel que l'amorolfine, un azole tel que le bifonazole, le clotrimazole, l'éconazole, le miconazole, l'oxiconazole, le croconazole, le fenticonazole, le tioconazole, le kétoconazole ou l'isoconazole, ou un composé allylique tel que la terbinafine ou la naftifine, la griséofulvine, le tolciclate, le tolnaftate ou la buténafine.

7. Vernis à ongles selon la revendication 5 ou 6, contenant l'agent antimycosique à action locale en une proportion de 0,5 à 20 % en poids, de préférence de 2 à 5 % en poids.

8. Procédé pour la fabrication d'un vernis à ongles selon une ou plusieurs des revendications 1 à 7, caractérisé en ce que l'on mélange l'agent filmogène insoluble dans l'eau, sous forme dissoute, avec le composé à action vasodilatatrice et on y ajoute éventuellement l'agent antimycosique à action locale et d'autres additifs.

9. Utilisation du vernis à ongles selon une ou plusieurs des revendications 1 à 7, ou d'un vernis à ongles selon une ou plusieurs des revendications 1 à 7 contenant en tant que composé à action vasodilatatrice du vérapamil, pour la fabrication d'un médicament destiné au traitement de troubles de la croissance des ongles.
